(19) Europäisches Patentamt
European Patent Office
Office européen des brevets

(11) EP 2 092 837 B1

(12) # EUROPEAN PATENT SPECIFICATION

(45) Date of publication and mention of the grant of the patent:
**21.09.2011 Bulletin 2011/38**

(51) Int Cl.:
*A23L 1/30* $^{(2006.01)}$ *A61K 8/37* $^{(2006.01)}$
*A61K 8/97* $^{(2006.01)}$ *A61Q 19/02* $^{(2006.01)}$

(21) Application number: **09153249.9**

(22) Date of filing: **19.02.2009**

(54) **Compositions for skin whitening comprising (2Z, 8Z) - matricaria acid methyl ester**

Zusammensetzung zur Hautaufhellung mit (2Z, 8Z)-Matricariasäure-Methylester

Compositions pour le blanchiment de la peau comprenant un ester méthylique de l'acide (2Z, 8Z) - matricaria

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **25.02.2008 KR 20080016748**

(43) Date of publication of application:
**26.08.2009 Bulletin 2009/35**

(73) Proprietor: **INHA-INDUSTRY PARTNERSHIP INSTITUTE**
**Nam-ku**
**Incheon**
**402-751 (KR)**

(72) Inventors:
- **Kim, Eun-ki**
  **135-280, SEOUL (KR)**
- **Lee, Nam Ho**
  **690-122, JEJU-DO (KR)**
- **Luo, Lianhua**
  **402-751, INCHEON (KR)**

(74) Representative: **Office Freylinger**
**P.O. Box 48**
**8001 Strassen (LU)**

(56) References cited:
**WO-A1-03/080637 KR-B1- 100 602 684**
**US-A- 5 443 839 US-A- 6 084 080**

- **J. YUE, Z. LIN, D. WANG, H. SUN: "A Sesquiterpene and Other Constituents from Erigeron Breviscapus" PHYTOCHEMISTRY, vol. 36, no. 3, 1994, pages 717-719, XP002532840**
- **B. CHEN, B.-G. LI, G.-L. ZHANG: "A New Sesquiterpene Glucoside from Erigeron Breviscapus" NATURAL PRODUCT RESEARCH, vol. 17, no. 1, 2003, pages 37-40, XP009118437**
- **DATABASE WPI Section Ch, Week 200868 Thomson Scientific, London, GB; Class B02, AN 2008-L53390 XP002535410 & CN 101 125 155 A (UNIV SICHUAN) 20 February 2008 (2008-02-20)**
- **DATABASE WPI Section Ch, Week 200209 Thomson Scientific, London, GB; Class B05, AN 2002-062710 XP002535411 & CN 1 174 708 A (LEI L) 4 March 1998 (1998-03-04)**
- **E.-K. KIM ET AL.: "Depigmentation of Melanocytes by (2Z, 8Z)-Matricaria Acid Methyl Ester Isolated from Erigeron breviscapus" BIOL. PHARM. BULL., vol. 32, no. 6, 2009, pages 1091-1094, XP002532841**
- **DATABASE WPI Week 200676 Thomson Scientific, London, GB; AN 2006-728987 XP002535412 & CN 1 762 402 A (YUNNAN BAIYAO GROUP DALI PHARM CO LTD) 26 April 2006 (2006-04-26)**
- **Y. KIMURA, A. KOBAYASHI ET AL.: "Isolation and Identification of Two Nematicidal Substances from Roots of Erigeron philadelphicus L. and Nematicidal Activities of Their Related Compounds" AGRIC. BIOL. CHEM., vol. 45, no. 12, 1981, pages 2915-2917, XP009118899**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 092 837 B1

## Description

This application claims the benefit of Korea Application No. 10-2008-0016748 filed on February 25, 2008.

## BACKGROUND OF THE INVENTION

### 1. Field of the Invention

The present invention relates to a composition for skin whitening comprising matricaria acid methyl ester as an active ingredient.

### 2. Description of the Related Art

There is the old saying in Asia: "white face skin covers three faults". White face has been regarded as a beauty since long. So, to make face skin white, natural materials such as cucumber and rice extract have been used. And demand for skin whitening is still growing.

The most important pigment that determines skin color is melanin which is synthesized in melanosomes in melanocytes of basal layer of skin. Tyrosine is oxidized by the enzyme tyrosinase and then converted into DOPAquinone via DOPA and further produced as the copolymer melanin via DOPAchrome by auto-oxidation and enzyme reaction. The generated melanin is transferred to keratinocytes by melanosome. Then, melanin has been through keratosis process for 28 days and as a result it is exposed on skin and disappeared with keratin.

Melanin absorbs light energy of UV from sunlight so that it protects skin organs under dermis from damage by UV. Melanin captures oxygen free radicals generated in skin, so that it protects skin from foreign harmful factors. Human skin color is determined by the amount of melanin in skin cells. If melanin level is high, skin color is determined as brown or black. If melanin level is low, skin color is determined as white. If melanin is over-synthesized by sun, hormone change, inflammation or drug or if keratosis process is interrupted, pigment is accumulated to cause skin diseases including melasma and freckle or skin cancer.

There are diverse attempts to suppress melanin generation. For example, melanin generation has been inhibited by inhibiting tyrosinase synthesis or tyrosinase activity, reducing melanin, inhibiting photo-oxidation or promoting melanin elimination, etc. As tyrosinase inhibitors, kojic acid, arbutin and their derivatives have been developed, but these inhibitors have disadvantages of instability, side effects, unsatisfactory effect or efficiency.

Recent studies are focused on screening an active component for whitening among natural materials particularly among plants in addition to the conventional whitening agents. As a result, many plant extracts and herb extracts from *Mori cortex* (Japanese Laid-Open Patent Publication S 55-44375, S 64-26507, S 64-83009, H 1-25687, H 5-139950, Korean Patent Publication No 92-002109, and No 97-021273), *Glycyrrhiza uralensis* (Japanese Laid-Open Patent Publication S 60-214721, S 60-214728, S 63-23809, S 64-63506, H 1-149706, Korean Patent Publication No 92-002109, and No 97-025601), *Paeonia lactiflora* (Japanese Laid-Open Patent Publication S 61-246109, and Korean Patent Publication No 92-002111), *Cinnamomum cassia* (Japanese Laid-Open Patent Publication S 63-30403, and H 5-139954), *Sophora flavescens* (Japanese Laid-Open Patent Publication S 64-26507, and Korean Patent Publication No 92-002110), *Pueraria lobata* (Japanese Laid-Open Patent Publication S 60-214727, and S 64-16709)., *Angelica gigas* (Japanese Laid-Open Patent Publication S 56-92211, and H 4-26610), *Paeonia suffruticosa* (Japanese Laid-Open Patent Publication S 60-214721, S 61-50915, and S 61-246109), *Pinellia ternata* (Japanese Laid-Open Patent Publication H 2-207028, and Korean Patent Publication No 96-033442), and Aloe (Japanese Laid-Open Patent Publication S 52-44375, H 2-207030, and H 5-139950) have been confirmed to have melanin generation inhibitory effect by working on tyrosinase. However, these extracts also have problems of instability and color change. Therefore, it is disadvantageous to add them to cosmetics or medicines more than effective dose. Besides, their active components are not disclosed yet and their effects are still in doubt.

The present inventors have studied on active component of *Erigeron breviscapus* extract based on the previous patent (Korean Patent No 10-0602684) saying that *Erigeron breviscapus* extract has whitening effect. As a result, the inventors separated and purified the active component responsible for whitening and identified thereof and investigated its effect. At last, the present inventors completed this invention by confirming that the hexane fraction of the said extract and (2Z,8Z)-matricaria acid methyl ester separated from the fraction inhibited melanin generation.

## SUMMARY OF THE INVENTION

It is an object of the present invention to provide an external agent, cosmetic composition, for skin whitening comprising (2Z,8Z)-matricaria acid methyl ester isolated from the *Erigeron breviscapus* extract as an active ingredient. The object of the invention is achieved by a use as claimed in claim 1.

The present document discloses a composition for skin whitening comprising the organic solvent fraction of *Erigeron breviscapus* extract as an active ingredient.

The present document discloses an external agent for skin whitening comprising the organic solvent fraction as an active ingredient.

The present document also describes a cosmetic composition for skin whitening comprising the organic solvent fraction as an active ingredient.

The present document also discloses a health food for skin whitening comprising the organic solvent fraction as an active ingredient.

The present invention also provides a composition for skin whitening comprising (2Z,8Z)-matricaria acid methyl ester as an active ingredient.

The present invention also provides an external agent for skin whitening comprising (2Z,8Z)-matricaria acid methyl ester as an active ingredient.

The present invention also provides a cosmetic composition for skin whitening comprising (2Z,8Z)-matricaria acid methyl ester as an active ingredient.

The present document also discloses a health food for skin whitening comprising (2Z,8Z)-matricaria acid methyl ester as an active ingredient.

The active fraction of *Erigeron breviscapus* extract and (2Z,8Z)-matricaria acid methyl ester isolated from the fraction can inhibit melanin generation in melanocytes but have no cytotoxicity, so that they can be effectively used for skin whitening.

## BRIEF DESCRIPTION OF THE DRAWINGS

The application of the preferred embodiments of the present invention is best understood with reference to the accompanying drawings, wherein:

Figure 1 is a diagram illustrating the $^{1}$H-NMR spectrum of (2Z,8Z)-matricaria acid methyl ester,
Figure 2 is a diagram illustrating the $^{13}$C-NMR spectrum of (2Z,8Z)-matricaria acid methyl ester,
Figure 3 is a diagram illustrating the MS spectrum of (2Z,8Z)-matricaria acid methyl ester,
Figure 4 is a graph illustrating the melanin generation and cell survival rate after (2Z, 8Z)-matricaria acid methyl ester treatment,
Figure 5 is a graph illustrating the melanin generation and cell survival rate after hexane fraction treatment.

## DESCRIPTION OF THE PREFERRED EMBODIMENTS

Hereinafter, the present invention is described in detail.

The present document discloses a composition for skin whitening comprising the organic solvent fraction of *Erigeron breviscapus* extract as an active ingredient.

The organic solvent fraction is preferably hexane fraction, but not always limited thereto.

The present invention also provides a composition for skin whitening comprising (2Z,8Z)-matricaria acid methyl ester as an active ingredient.

The organic solvent fraction or (2Z,8Z)-matricaria acid methyl ester can be produced by the following steps:

1) extracting *Erigeron breviscapus* using water, alcohol or a mixture thereof;
2) preparing an organic solvent fraction by adding an organic solvent to the extract of step 1); and
3) obtaining an active compound from the fraction of step 2) by column chromatography.

In this method, the *Erigeron breviscapus* of step 1) can be cultivated or purchased and herein whole part of the plant is preferably used.

The extraction solvent herein is preferably water, alcohol or a mixture thereof. The said alcohol is preferably $C_1$ - $C_4$ lower alcohol and the lower alcohol is preferably methanol, but not always limited thereto.

The methanol is preferably added 5-15 times the volume of dried *Erigeron breviscapus* and more preferably added 5-7 times the volume of dried *Erigeron breviscapus.* Extraction temperature is preferably 30 - 70°C and more preferably 40 - 50°C, but not always limited thereto. Extraction time is preferably 5-15 hours and more preferably 10 hours, but not always limited thereto. Extraction is preferably performed 1-5 times and more preferably 3 times repeatedly, but not always limited thereto.

In this method, the organic solvent of step 2) is preferably hexane and the preferable volume of the solvent is 1 - 3 times the volume of *Erigeron breviscapus* extract and double the volume of *Erigeron breviscapus* extract is more preferred, but not always limited thereto.

Whole plant of *Erigeron breviscapus* was pulverized, to which methanol was added 4 times the volume of the pulverized *Erigeron breviscapus,* followed by heating in a 40°C water bath for 10 hours. The extract was cooled down at room temperature and filtered with a filter paper to give a filtrate. The obtained filtrate was concentrated under reduced pressure to give an extract. The prepared *Erigeron breviscapus* extract was dissolved in water, to which equal volume of hexane was added, followed by partition to give hexane layer.

In this method, the column chromatograph of step 3) is performed using one of fillers selected from the group consisting of silicagel, sephadex, RP-18, polyamide, Toyopearl and XAD resin to separate an active compound. The column chromatography can be repeated several times with different fillers properly selected each time and hexane-chloroform can be used as a solvent at this time, but not always limited thereto.

The hexane fraction was absorbed on the silicagel column, followed by separation of an active compound with hexane-chloroform density gradient (hexane:chloroform = 100%:0% - 0%:100%). As a result, an active compound was separated at the concentration ratio of 55%:455%. The structure of the separated compound was analyzed by nuclear magnetic resonance (NMR). The compound was identified as (2Z,8Z)-matricaria acid methyl ester represented by formula 1.

【Formula 1】

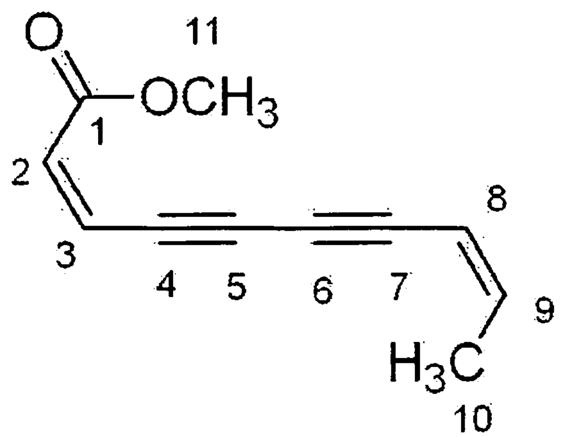

The organic solvent fraction of *Erigeron breviscapus* or of (2Z,8Z)-matricaria acid methyl ester isolated therefrom of the present invention is characterized by inhibiting melanin generation.

To investigate whitening effect, the present inventors cultured melan-A cells, which are melanocytes, and treated the hexane fraction and (2Z,8Z)-matricaria acid methyl ester of the present invention thereto. Then, melanin level was investigated by measuring OD. As a result, the hexane fraction inhibited melanin generation at a lower concentration, compared with the non-treated negative control (see Table 1 and Figure 5). The (2Z,8Z)-matricaria acid methyl ester also inhibited melanin generation at a lower concentration, compared with the positive control phenylthiourea (PTU, the known melanin generation inhibitor) and the non-treated negative control (see Table 1 and Figure 4). Therefore, it was confirmed that the organic solvent fraction and (2Z,8Z)-matricaria acid methyl ester inhibited melanin generation, suggesting that they have whitening effect.

The present inventors performed cytotoxicity test in order to investigate whether the organic solvent fraction and (2Z,8Z)-matricaria acid methyl ester could be used for skin whitening. Cells were inoculated in a well plate, to which the hexane fraction and (2Z,8Z)-matricaria acid methyl ester were treated at different concentrations, followed by culture and staining. Then, OD was measured. As a result, the hexane fraction and (2Z,8Z)-matricaria acid methyl ester treated cells demonstrated high survival rate at a low concentration, suggesting that no cytotoxicity was observed at a low concentration of each hexane, fraction and (2Z,8Z)-matricaria acid methyl ester (see Table 2, Figures 4 and 5). Therefore, the organic solvent fraction and (2Z,8Z)-matricaria acid methyl ester were confirmed to have no cytotoxicity at a low concentration but have high melanin generation inhibitory effect.

The composition of the present invention can include, in addition to (2Z,8Z)-matricaria acid methyl ester, one or more effective ingredients having the same or similar function to (2Z,8Z)-matricaria acid methyl ester.

The organic solvent fraction or (2Z,8Z)-matricaria acid methyl ester can be administered orally or parenterally

and be used in general forms. The pharmaceutical formulation is not a part of the invention.

The organic solvent fraction or (2Z,8Z)-matricaria acid methyl ester can be prepared for oral or parenteral administration by mixing with one or more pharmaceutically acceptable, generally used carriers or additives. In this case, any pharmaceutically acceptable carrier or additive can be used. Particularly, lactose monohydrate, cornstarch, soybean oil, microcrystalline cellulose or D-mannitol can be used as a diluent. Magnesium stearate or talc can be used as a lubricant. Polyvinylpyrolidone (PVP) or hydroxypropylcellulose (HPC) can be used as a binder. Carboxymethylcellulose calcium (Ca-CMC), sodium starch glycolate, polacrylin potassium or cross-linked

polyvinylpyrrolidone can be used as a disintegrating agent. White sugar, fructose, sorbitol or aspartame can be used as a sweetening agent. Carboxymethylcellulose sodium (Ma-CMC), betacyclodextrin, white bee's wax or xanthan gum can be used as a stabilizing agent. Methyl p-hydroxy benzoate (methylparaben), propyl p-hydroxy benzoate (propylparaben) or potassium sorbate can be used as an antiseptic. The organic solvent fraction or (2Z,8Z)-matricaria acid methyl ester are not limited in the types of formulations and can be prepared as compositions for beverages, powders, capsules, soft capsules, tablets, gums, viscous liquids; preparations for transdermal administration such as lotions, ointments, gels, creams, patches and aerosols; and formulations such as extracts, pills, tablets, powders and granules.

The dosage unit can contain, for example, 1, 2, 3 or 4 individual doses or 1/2, 1/3 or 1/4 of an individual dose. An individual dose preferably contains the amount of active compound which is administered in one application and which usually corresponds to a whole, 1/2, 1/3 or 1/4 of a daily dose. The effective dosage of the composition is preferably 0.00001 - 10 weight%,

and more preferably 0.0001 - 1 weight%. But the dosage can be increased or decreased according to the purpose of use as long as whitening effect is secured without toxicity.

The present invention also provides a skin whitening agent for external application comprising (2Z,8Z)-matricaria acid methyl ester as an active ingredient.

The preparation for skin external application containing (2Z,8Z)-matricaria acid methyl ester isolated from the organic solvent fraction of *Erigeron breviscapus* extract of the present invention can additionally include a supplement generally used in the field of skin science such as fatty substance, organic solvent, resolvent, concentrate, gelling agent, softener, antioxidant, suspending agent, stabilizer, foaming agent, odorant, surfactant, water, ionic or non-ionic emulsifying agent, filler, sequestering agent, chelating agent, preserving agent, vitamin, blocker, moisturing agent, essential oil, dye, pigment, hydrophilic or hydrophobic activator, lipid vesicle or other components generally used in a preparation for skin external application. The amount of the above supplement can be determined as generally accepted in the field of skin science.

The present invention also provides a cosmetic composition comprising (2Z,8Z)-matricaria acid methyl ester as an active ingredient.

For the cosmetic composition used in the present invention, the (2Z,8Z)-matricaria acid methyl ester is preferably added 0.005 - 80 weight part by the total composition weight but the content can be increased or decreased according to the purpose of use as long as whitening effect is secured without toxicity.

The cosmetic composition of the present invention can include, in addition to (2Z,8Z)-matricaria acid methyl ester, one or more effective ingredients having the same or similar function to (2Z,8Z)-matricaria acid methyl ester. The cosmetics containing (2Z,8Z)-matricaria acid methyl ester of the present invention as an active ingredient can be formulated as general emulsified cosmetics solubilized cosmetics. The emulsified cosmetics are exemplified by lotion, cream and essence, and the solubilized cosmetics are exemplified by skin. In addition, it can be formulated as a supplement for local or systemic application generally used in the field of skin science by containing skin scientifically acceptable medium or base.

The proper formulation of the cosmetics can be exemplified by solution, gel, solid or dough anhydride, emulsion prepared by dispersing oil phase on water phase, suspension, microemulsion, microcapsule, microgranule, ionic (liposome) or non-ionic vesicle, cream, skin, lotion powder, spray, and conceal stick. In addition, the cosmetics can be formulated as an aerosol composition containing foam or compressed propellant.

The cosmetic composition for whitening of the present invention can include, in addition to (2Z,8Z)-matricaria acid methyl ester of the present invention, a supplement generally used in the field of cosmetics such as fatty substance, organic solvent, resolvent, concentrate, gelling agent, softener, antioxidant, suspending agent, stabilizer, foaming agent, odorant, surfactant, water, ionic or non-ionic emulsifying agent, filler, sequestering agent, chelating agent, preserving agent, vitamin, blocker, moisturing agent, essential oil, dye, pigment, hydrophilic or hydrophobic activator, lipid vesicle or other components generally used in cosmetics.

The acceptable carrier for the cosmetic composition for skin whitening of the present invention can be properly selected according to the type of formulation which is exemplified by solution, suspension, emulsion, paste, gel, cream, lotion, soap, shampoo, surfactant-containing cleansing, oil, powdered foundation, liquid foundation, cream foundation, and spray, and more preferably soft lotion, nutrition lotion, nutrition cream, massage cream, essence, eye cream, cleansing cream, cleansing foam, cleansing water, pack, and hair essence.

In the case that the cosmetic composition is formulated as paste, cream or gel, the proper carrier is preferably

selected from the group consisting of animal oil, vegetable oil, paraffin, starch, trakind, cellulose derivative, polyethylene glycol, silicon, bentonite, silica, talk and zinc oxide.

In the case that the cosmetic composition is formulated as solution or emulsion, the proper carrier is preferably selected from the group consisting of solvents, solvating agents and emulsifying agents, which are exemplified by water, ethanol, isopropanol, ethyl carbonate, ethyl acetate, benzene alcohol, benzene benzoate, propylene glycol, 1,3-butylglycol oil, glycerol aliphatic ester, polyethylene glycol and sorbitan aliphatic ester.

In the case that the cosmetic composition is formulated as suspension, the proper carrier is preferably selected from the group consisting of liquid diluents such as water, ethanol or propylene glycol, ethoxylated isostearyl alcohol, aluminum methahydroxide, bentonite, agar and tragacanth.

In the case that the cosmetic composition is formulated as surfactant-containing cleansing, the proper carrier is preferably selected from the group consisting of aliphatic alcohol sulfate, aliphatic alcohol ether sulfate, sulphosuccinic acid monoester, isethionate, imidazolinium derivative, methyl taurate, sarcosinate, fatty acid amide ether sulfate, alkylamidibetain, aliphatic alcohol, fatty acid glyceride, fatty acid diethanolamide, vegetable oil, ranoline derivative and ethoxylated glycerol fatty acid ester.

The present document also discloses a health food for skin whitening comprising the organic solvent fraction or (2Z,8Z)-matricaria acid methyl ester as an active ingredient.

In that case, the organic solvent fraction or (2Z,8Z)-matricaria acid methyl ester can be added as it is or as mixed with other food components according to the conventional method. The mixing ratio of active ingredients can be regulated according to the purpose of use (prevention, health enhancement or treatment). In general, to produce health food or beverages, the organic solvent fraction or (2Z,8Z)-matricaria acid methyl ester is added preferably up to 15 weight part and more preferably up to 10 weight part. However, if long term administration is required for health and hygiene or regulating health condition, the content can be lower than the above but higher content can be accepted as well since the organic solvent fraction or (2Z,8Z)-matricaria acid methyl ester has been proved to be very safe.

The food herein is not limited. For example, the organic solvent fraction or (2Z,8Z)-matricaria acid methyl ester can be added to meat, sausages, bread, chocolates, candies, snacks, cookies, pizza, ramyuns, flour products, gums, dairy products including ice cream, soups, beverages, tea, drinks, alcohol drinks and vitamin complex, etc, and in wide sense, almost every food applicable in the production of health food can be included.

The composition for health beverages of the present invention can additionally include various flavors or natural carbohydrates, etc, like other beverages. The natural carbohydrates above can be one of monosaccharides such as glucose and fructose, disaccharides such as maltose and sucrose, polysaccharides such as dextrin and cyclodextrin, and glucose alcohols such as xylitol, sorbitol and erythritol. Besides, natural sweetening agents such as thaumatin and stevia extract, and synthetic sweetening agents such as saccharin and aspartame can be included. The content of the natural carbohydrate is preferably 0.01-0.04 g and more preferably 0.02-0.03 g in 100 mℓ of the composition.

In addition to the ingredients mentioned above, the organic solvent fraction or (2Z,8Z)-matricaria acid methyl ester can include in variety of nutrients, vitamins, minerals, flavors, coloring agents, pectic acid and its salts, alginic acid and its salts, organic acid, protective colloidal viscosifiers, pH regulators, stabilizers, antiseptics, glycerin, alcohols, carbonators which used to be added to soda, etc. The organic solvent fraction or (2Z, 8Z) -matricaria acid methyl ester can also include natural fruit juice, fruit beverages and/or fruit flesh addable to vegetable beverages. All the mentioned ingredients can be added singly or together. The mixing ratio of those ingredients does not matter in fact, but in general, each can be added by 001-0.1 weight part per 100 weight part of the organic solvent fraction or (2Z,8Z)-matricaria acid methyl ester.

Practical and presently preferred embodiments of the present invention are illustrative as shown in the following Examples, Experimental Examples and Manufacturing Examples.

<u>Example 1: Preparation of hexane fraction and (2Z,8Z)-matricaria acid methyl ester</u>

70 g of *Erigeron breviscapus* whole plant collected in alpine region, China, was pulverized, to which 400 mℓ of methanol was added, followed by heating in a 40°C water bath for 10 hours. The extract was cooled down at room temperature and filtered with a filter paper to give a filtrate. The obtained filtrate was concentrated under reduced pressure to give 14 g of the extract. The prepared *Erigeron breviscapus* extract was dissolved in 400 mℓ of water, to which equal volume of hexane was added, followed by partition to give a hexane layer. Chromatography was performed with 1 g of the hexane fraction obtained after evaporation using silicagel filled column. At this time, a mixed solution of hexane and chloroform was used as a solvent and hexane-chloroform density gradient (hexane:chloroform = 100%:0% → 0%:100%) was used. As a result, 117.5 mg of (2Z,8Z)-matricaria acid methyl ester was obtained at the concentration ratio of 55%: 45%.

Example 2: Structure analysis

To identify the structure of (2Z,8Z)-matricaria acid methyl ester obtained in Example 1, instrumental analysis was performed. For $^1$H and $^{13}$C-NMR, VARIAN UNITYINOVA400 (Varian Technology) was used. At this time, the sample was dissolved in dimethyl-d6 sulfoxide 99.9 atom % D. For GS-MS, Varian 1200L Single Quadrupole GC/MS System with 3800GC (Varian Technology) was used. The results are shown in the below (Figures 1, 2 and 3).
$^1$H NMR(DMSO-$d_6$, 400MHz) : δ 6.56 (1H, d, $J$ = 11.6, H-3), 6.40(1H, d, $J$ = 11.6, H-2), 6.36(1H, dq, $J$ = 10.8, 6.8, H-9), 5.78(1H, dq, $J$ = 10.8, 1.2, H-8) , 3.68(3H, s, H-11) , 1.88(3H, dd, $J$ = 6.8, 1.2, H-10);
$^{13}$C NMR(DMSO-$d_6$, 100MHz): δ 163.9(C-1), 145.1(C-9), 131.6(C-3), 122.1(C-2), 108.4(C-8), 84.2(C-5), 83.4(C-7), 78.7 (C-6), 77.3(C-4), 51.5(C-11), 16.6(C-10) ;
HMBC correlations(H-C#) H-2→C-1; H-3→C-4, C-5; H-10→C-7, C-8, C-9; H-11→C-1; and
EIMS: *m/z* 174 ($M^+$; rel int 40), 159 (M-$CH_3$, 35), 143 (M-$OCH_3$, 20), 115(M-$CH_3CO_2$, 35), 103(100).

Experimental Example 1: Whitening effect

<1-1> Melan-A melanocyte culture

Melan-A melanocytes were provided by AmorePacific Co., Korea. Melan-A is the cell line producing melanin, which has been usually used for the examination of a whitening agent. For cell culture, RPMI1640 medium supplemented with 10% FBS, 1% penicillin/streptomycin, and 200 nM TPA was prepared and sterilized. The cells were cultured in a 37°C, 10% $CO_2$ incubator. When the melan-A cells were grown to fill 90% of 100 mm Petri-dish, the cells were subcultured.

<1-2> Investigation of melanin generation inhibition by measuring OD

To investigate whitening effect, melan-A cells were treated with the hexane fraction, (2Z,8Z)-matricaria acid methyl ester and phenylthiourea, followed by measuring melanin levels. Particularly, the cells were seeded in a 6-well plate, to which 6 ug/ml of the hexane fraction, 4.5 uM and 9 uM of (2Z,8Z)-matricaria acid methyl ester, and 50 uM of phenylthiourea were treated, followed by culture for three days. The cells were recovered by using trypsine-EDTA. 1 N NaOH containing 10% DMSO (dimethylsulfoxide) was added to the recovered cells, followed by reaction at 80°C for 1 hour to let melanin dissolved fully. Then, $OD_{405}$ was measured using ELISA reader (VERSAmax, Molecular device, USA). Melanin generation was quantified by the following mathematical formula 1 (Table 1, Figures 4 and 5).

【Mathematical Formula 1】

$$\text{Melanin generation rate (\%)} = \text{OD of treated group} / \text{OD of non-treated group} \times 100$$

[Table 1]

| Sample | Melanin generation rate (%) |
|---|---|
| Non-treated group | 100 |
| Phenylthiourea (50 uM) | 36.19 |
| Hexane fraction (6 ug/ml) | 61.5 |
| (2Z,8Z)-matricaria acid methyl ester (4.5 uM) | 36.37 |
| (2Z,8Z)-matricaria acid methyl ester (9 uM) | 26.92 |

As a result, as shown in Table 1, when phenylthiourea known as a melanin generation inhibitor was treated at the concentration of 50 uM, melanin generation rate was 36.19%. When the hexane fraction was treated at the concentration of 6 ug/ml, melanin generation rate was 61.50%. In the meantime, when (2Z,8Z)-matricaria acid methyl ester was treated at the concentrations of 4.5 uM and 9 uM, melanin generation rates were 36.37% and 26.92%, suggesting that melanin generation inhibitory effect was increased dose-dependently and the hexane fraction demonstrated melanin

inhibitory effect even at much lower concentration.

Experimental Example 2: Toxicity test

To confirm whether the hexane fraction and (2Z,8Z)-matricaria acid methyl ester could be used as a cosmetic composition for skin whitening, the present inventors performed cytotoxicity test. Melan-A cells were seeded in a 96-well plate, to which 6 ug/ml of the hexane fraction and 4.5 uM an 9 uM of (2Z,8Z)-matricaria acid methyl ester were treated, followed by culture in a 37°C, 10% $CO_2$ incubator for three days. Upon completion of the culture, the medium was eliminated and 100 $\mu\ell$ of MTT (3-(4,5-dimethythiazol-2-yl)-2,5-diphenyltetrazoliu, bromide) solution was added to each well of the plate, followed by reaction at 37°C for 4 hours. Upon completion of the reaction, the solution was eliminated and 100 $\mu\ell$ of DMSO was added to each well of the plate to lyse the stained cells. Then, $OD_{540}$ was measured using ELISA reader. Cell survival rate was calculated by the following mathematical formula 2 (Table 2, Figures 4 and 5).

【Mathematical Formula 2】

$$\text{Cell survival rate (\%)} = (\text{OD of treated group} / \text{OD of non-treated group}) \times 100$$

[Table 2]

| Sample | Cell survival rate (%) |
|---|---|
| Non-treated group | 100 |
| Phenylthiourea (50 uM) | 100.19 |
| Hexane fraction (6 ug/ml) | 100.6 |
| (2Z,8Z)-matricaria acid methyl ester (4.5 uM) | 106.66 |
| (2Z,8Z)-matricaria acid methyl ester (9 uM) | 99.44 |

As a result, as shown in Table 2, when 6 ug/ml of the hexane fraction was treated, cell survival rate was 100.6%, compared with the non-treated group, and when (2Z,8Z)-matricaria acid methyl ester was treated at the concentrations of 4.5 uM and 9 uM respectively, cell survival rates were 106.66% and 99.44%, indicating that these substances had melanin generation inhibitory effect without toxicity.

The Manufacturing Examples of the compound of formula 1 are described hereinafter.

Manufacturing Example 1: Preparation of powders

| | |
|---|---|
| Compound of formula 1 | 20 mg |
| Lactose | 100 mg |
| Talc | 10 mg |

Powders were prepared by mixing all the above components, which were filled in airtight packs according to the conventional method for preparing powders.

Manufacturing Example 2: Preparation of tablets

| | |
|---|---|
| Compound of formula 1 | 10 mg |
| Corn starch | 100 mg |
| Lactose | 100 mg |

(continued)

| | |
|---|---|
| Magnesium stearate | 2 mg |

Tablets were prepared by mixing all the above components by the conventional method for preparing tablets.

Manufacturing Example 3: Preparation of powders

| | |
|---|---|
| Compound of formula 1 | 10 mg |
| Crystalline cellulose | 3 mg |
| Lactose | 14.8 mg |
| Magnesium stearate | 0.2 mg |

Capsules were prepared by mixing all the above components, which were filled in gelatin capsules according to the conventional method for preparing capsules.

Manufacturing Example 4: Preparation of injections

| | |
|---|---|
| Compound of formula 1 | 10 mg |
| Mannitol | 180 mg |
| Sterilized DW for injection | 2974 mg |
| $Na_2HPO_4,12H_2O$ | 26 mg |

Injections were prepared by mixing all the above components, putting the mixture into 2 mℓ ampoules and sterilizing thereof by the conventional method for preparing injections.

Manufacturing Example 5: Preparation of liquid formulations

| | |
|---|---|
| Compound of formula 1 | 20 mg |
| Isomerized sugar | 10 g |
| Mannitol | 5 g |
| Purified water | proper amount |

Liquid formulations were prepared by mixing all the above components, putting the mixture into brown bottles and sterilizing thereof by the conventional method for preparing liquid formulations.

Manufacturing Example 6: Preparation of health food

| | |
|---|---|
| Compound of formula 1 | 1000 mg |
| Vitamin complex | proper amount |
| Vitamin A acetate | 70 μg |
| Vitamin E | 1.0 mg |
| Vitamin B1 | 0.13 mg |
| Vitamin B2 | 0.15 mg |
| Vitamin B6 | 0.5 mg |
| Vitamin B12 | 0.2 μg |

(continued)

| | |
|---|---|
| Vitamin C | 10 mg |
| Biotin | 10 μg |
| Nicotinic acid amide | 1.7 mg |
| Folic acid | 50 μg |
| Calcium pantothenate | 0.5 mg |
| Minerals | proper amount |
| Ferrous sulfate | 1.75 mg |
| Zinc oxide | 0.82 mg |
| Magnesium carbonate | 25.3 mg |
| Potassium phosphate monobasic | 15 mg |
| Potassium phosphate dibasic | 55 mg |
| Potassium citrate | 90 mg |
| Calcium carbonate | 100 mg |
| Magnesium chloride | 24.8 mg |

Vitamins and minerals were mixed according to the preferable composition rate for health food. However, the composition rate can be adjusted. The constituents were mixed according to the conventional method for preparing health food and then the composition for health food (ex. health candies, etc) was prepared according to the conventional method.

Manufacturing Example 7: Preparation of health beverages

| | |
|---|---|
| Compound of formula 1 | 1000 mg |
| Citric acid | 1000 mg |
| Oligosaccharide | 100 g |
| Maesil (Prunus mume) Extract | 2 g |
| Taurine | 1 g |
| Purified water | up to 900 mℓ |

The above constituents were mixed according to the conventional method for preparing health beverages. The mixture was heated at 85°C for 1 hour with stirring and then filtered. The filtrate was loaded in 2 liter sterilized containers, which were sealed and sterilized again, stored in a refrigerator until they would be used for the preparation of a composition for health beverages.

The constituents appropriate for favorite beverages were mixed according to the preferred mixing ratio but the composition ratio can be adjusted according to regional and national preferences, etc.

Manufacturing Example 8: Preparation of cosmetics for skin whitening

<8-1> Preparation of skin lotion

Skin lotion containing the compound of formula 1 as an active ingredient was prepared according to the composition shown in Table 3.

[Table 3]

| Raw material | Content (weight part) |
|---|---|
| (2Z,8Z)-matricaria acid methyl ester | 0.0005 |
| 1,3-butyleneglycol | 1.00 |
| Disodium EDTA | 0.05 |
| Allantoin | 0.10 |

(continued)

| Raw material | Content (weight part) |
|---|---|
| Dipotassium glycyrrhizinate | 0.05 |
| Citric acid | 0.01 |
| Sodium citrate | 0.02 |
| Glycereth-26 | 1.00 |
| Arbutin | 2.00 |
| Hydrogenated castor oil | 1.00 |
| Ethanol | 30.00 |
| Preservative | Small amount |
| Colorant | Small amount |
| Flavor | Small amount |
| Purified water | Small amount |

<8-2> Preparation of nutrition cream

Nutrition cream containing the compound of formula 1 as an active ingredient was prepared according to the composition shown in Table 4.

[Table 4]

| Raw material | Content (weight part) |
|---|---|
| (2Z,8Z)-matricaria acid methyl ester | 0.0005 |
| 1,3-butyleneglycol | 7.0 |
| Glycerin | 1.0 |
| D-panthenol | 0.1 |
| Plant extract | 3.2 |
| Magnesium aluminum silicate | 0.3 |
| PEG-40 stearate | 1.2 |
| Stearic acid | 2.0 |
| Polysorvate 60 | 1.5 |
| Lipophilic glyceryl stearate | 2.0 |
| Sorbitan sesquioleate | 1.5 |
| Cetearyl alcohol | 3.0 |
| Mineral oil | 4.0 |
| Squalane | 3.8 |
| Caprylic/Capric triglyceride | 2.8 |
| Vegitable oil | 1.8 |
| Dimethicone | 0.4 |
| Dipotassium glycyrrhizinate | Small amount |
| Allantoin | Small amount |
| Sodium hyaluronate | Small amount |
| Tocopheryl acetate | Proper amount |

(continued)

| Raw material | Content (weight part) |
|---|---|
| Triethanolamine | Proper amount |
| Preservative | Proper amount |
| colorant | Proper amount |
| Purified water | Proper amount |

Those skilled in the art will appreciate that the conceptions and specific embodiments disclosed in the foregoing description may be readily utilized as a basis for modifying or designing other embodiments for carrying out the same purposes of the present invention.

## Claims

1. Use of (2Z,8Z)-matricaria acid methyl ester for skin whitening.

2. The use according to claim 1, wherein (2Z,8Z)-matricaria acid methyl ester inhibits melanin generation.

3. The use according to claim 1, wherein the (2Z,8Z)-matricaria acid methyl ester is comprised in a cosmetic composition as an active ingredient.

4. The use according to claim 3, wherein the cosmetic composition is a composition for the skin external application.

5. The use according to claims 3 and 4, wherein the cosmetic composition comprises (2Z,8Z)-matricaria acid methyl ester from 0.005 to 80 weight part by the cosmetic composition weight.

## Patentansprüche

1. Verwendung von (2Z,BZ)-Matricariasäure-methylester zur Hautaufhellung.

2. Verwendung nach Anspruch 1, wobei der (2Z,SZ)-Matricariasäure-methylester die Melaninbildung hemmt.

3. Verwendung nach Anspruch 1, wobei der (2Z,BZ)-Matricarissaure-methylester in einer kosmetischen Zusammensetzung als Wirkstoff umfasst ist.

4. Verwendung nach Anspruch 3, wobei die kosmetische Zusammensetzung eine Zusammensetzung zur äußerliche Anwendung auf der Haut ist.

5. Verwendung nach Anspruch 3 und 4, wobei die kosmetische Zusammensetzung (2Z,BZ)-Matricariasäure-methylester zu 0,005 bis 80 Gewichtstellen nach dem Gewicht der kosmetischen Zusammensetzung umfasst.

## Revendications

1. Utilisation de l'ester méthylique de l'acide (2Z,8Z)-matricaria pour le blanchiment de la peau.

2. Utilisation selon la revendication 1, dans laquelle l'ester méthylique de l'acide (2Z,8Z)-matricaria inhibe la génération de mélanine.

3. Utilisation selon la revendication 1, dans laquelle l'ester méthylique de l'acide (2Z,8Z)-matricaria est compris dans une composition cosmétique à titre d'ingrédient actif.

4. Utilisation selon la revendication 3, dans laquelle la composition cosmétique est une composition pour l'application cutanée externe.

5. Utilisation selon les revendications 3 et 4, dans laquelle la composition cosmétique comprend l'ester méthylique de l'acide (2Z,8Z)-matricaria à concurrence de 0,005 à 80 parties en poids par rapport au poids de la composition cosmétique.

FIGURE 1

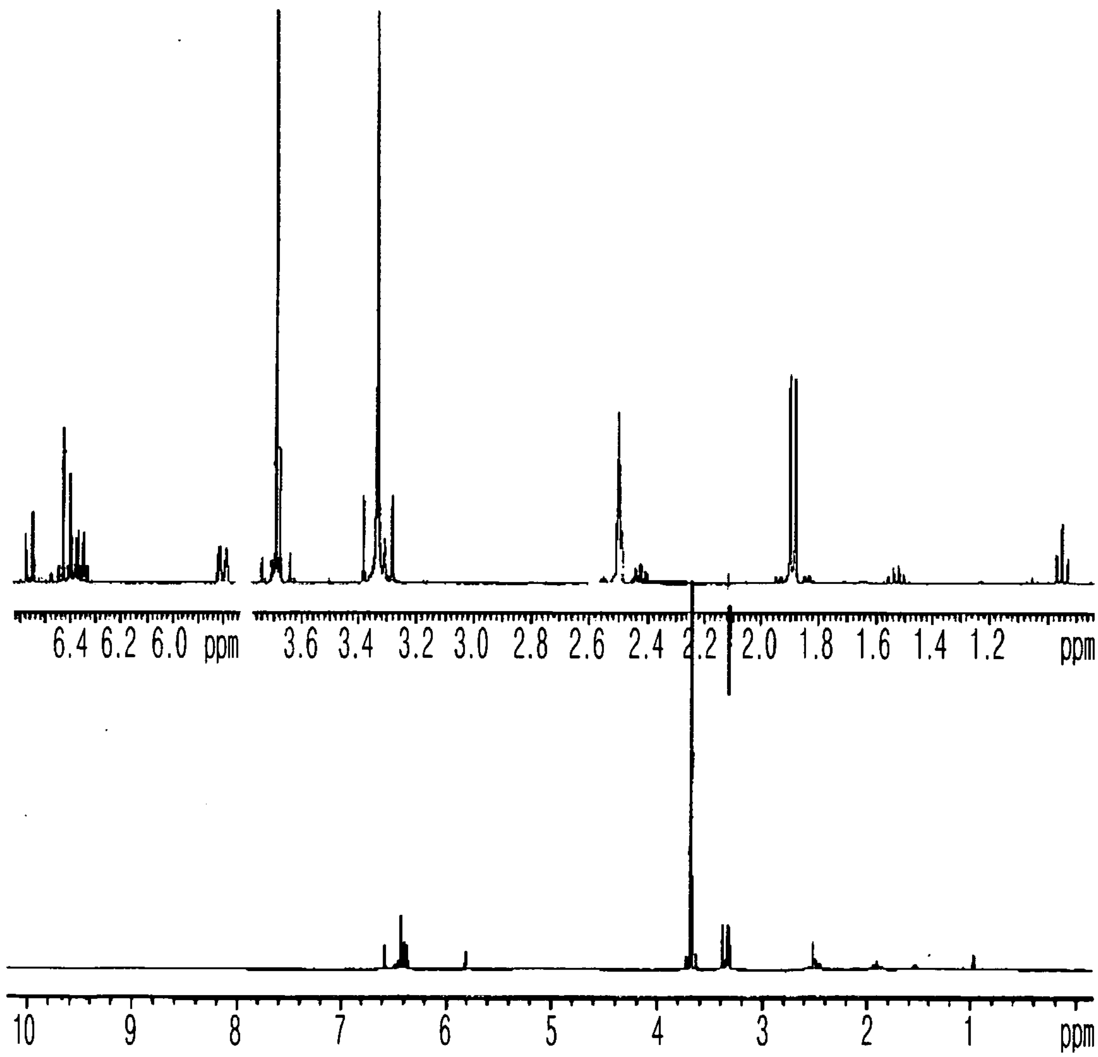
6.4 6.2 6.0 ppm
3.6 3.4 3.2 3.0 2.8 2.6 2.4 2.2 2.0 1.8 1.6 1.4 1.2 ppm
10 9 8 7 6 5 4 3 2 1 ppm

FIGURE 2

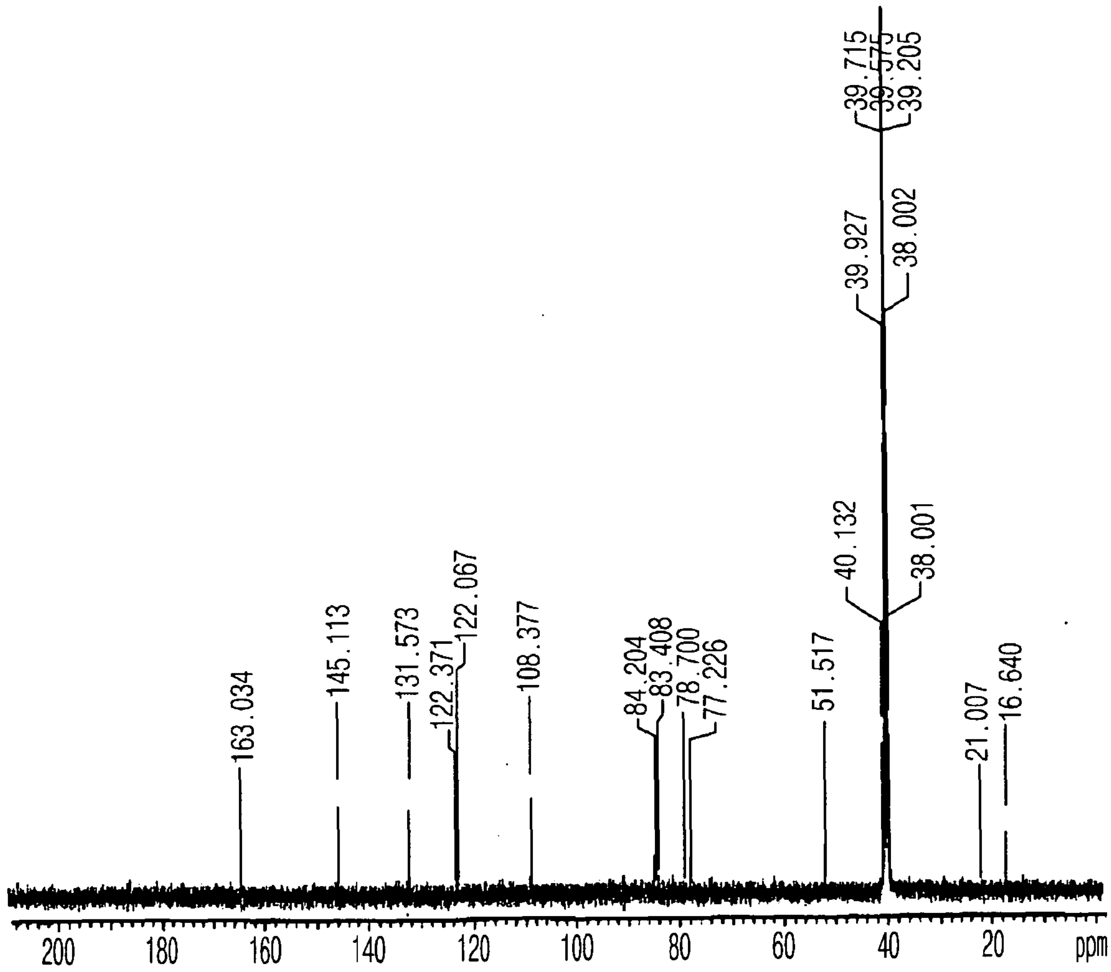
39.715
39.575
39.205
39.927
38.002
40.132
38.001
163.034
145.113
131.573
122.371
122.067
108.377
84.204
83.408
78.700
77.226
51.517
21.007
16.640
200
180
160
140
120
100
80
60
40
20
ppm

FIGURE 3

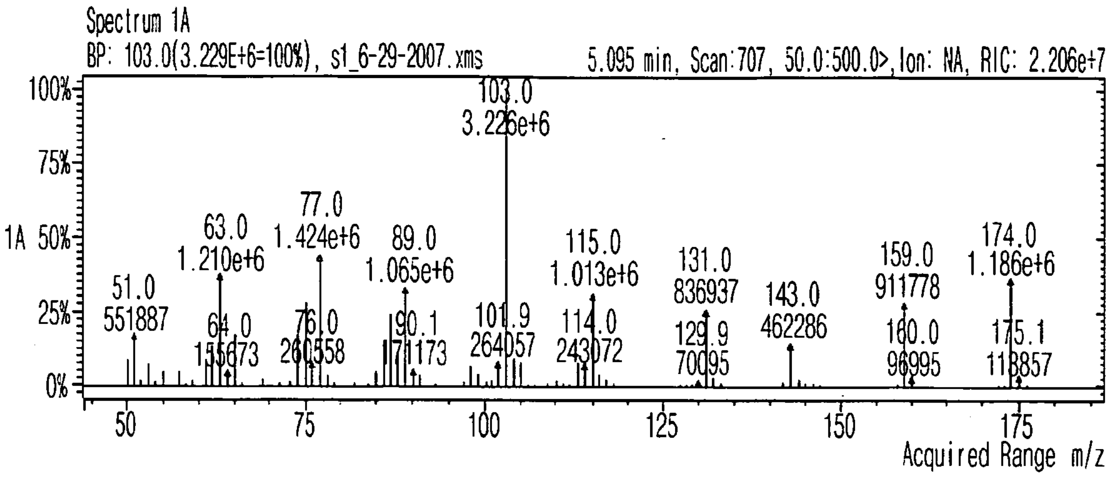
Spectrum 1A
BP: 103.0(3.229E+6=100%), s1_6-29-2007.xms
5.095 min, Scan:707, 50.0:500.0>, Ion: NA, RIC: 2.206e+7
100%
75%
1A 50%
25%
0%
103.0
3.226e+6
77.0
1.424e+6
63.0
1.210e+6
89.0
1.065e+6
115.0
1.013e+6
131.0
836937
159.0
911778
174.0
1.186e+6
51.0
551887
143.0
462286
64.0
155673
76.0
260558
90.1
171173
101.9
264057
114.0
243072
129.9
70095
160.0
96995
175.1
118857
50
75
100
125
150
175
Acquired Range m/z

FIGURE 4

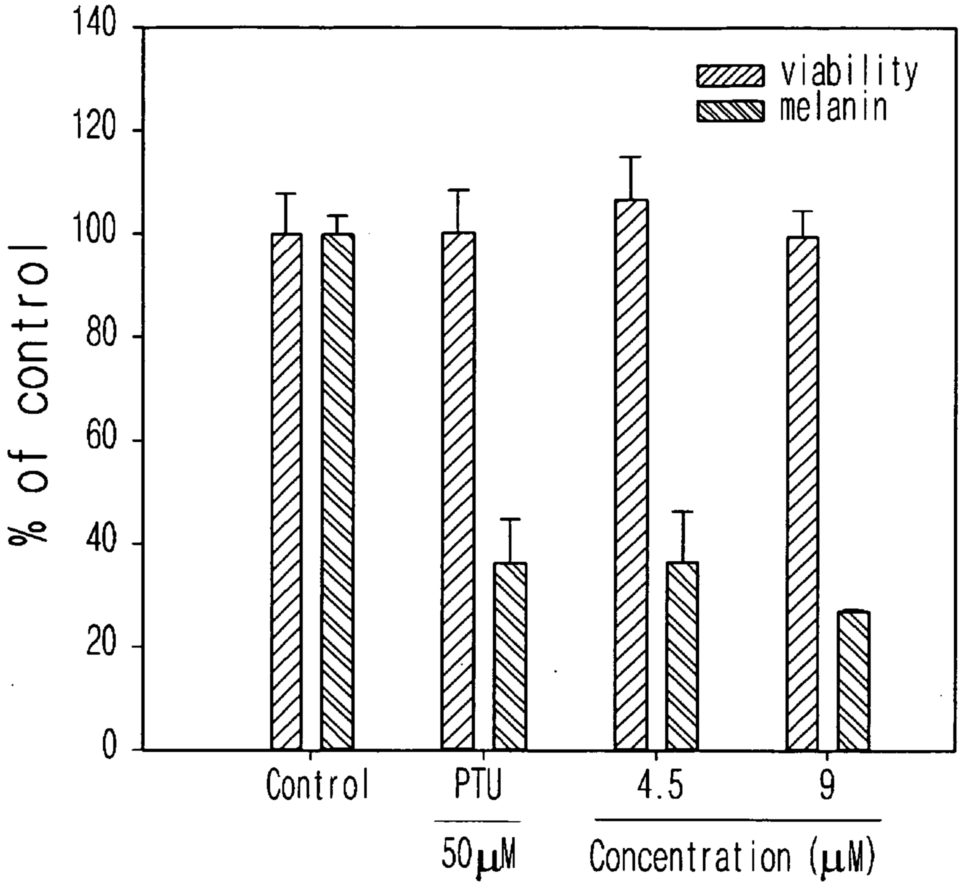
140
120
100
80
60
40
20
0
% of control
viability
melanin
Control
PTU
4.5
9
50μM
Concentration (μM)

FIGURE 5

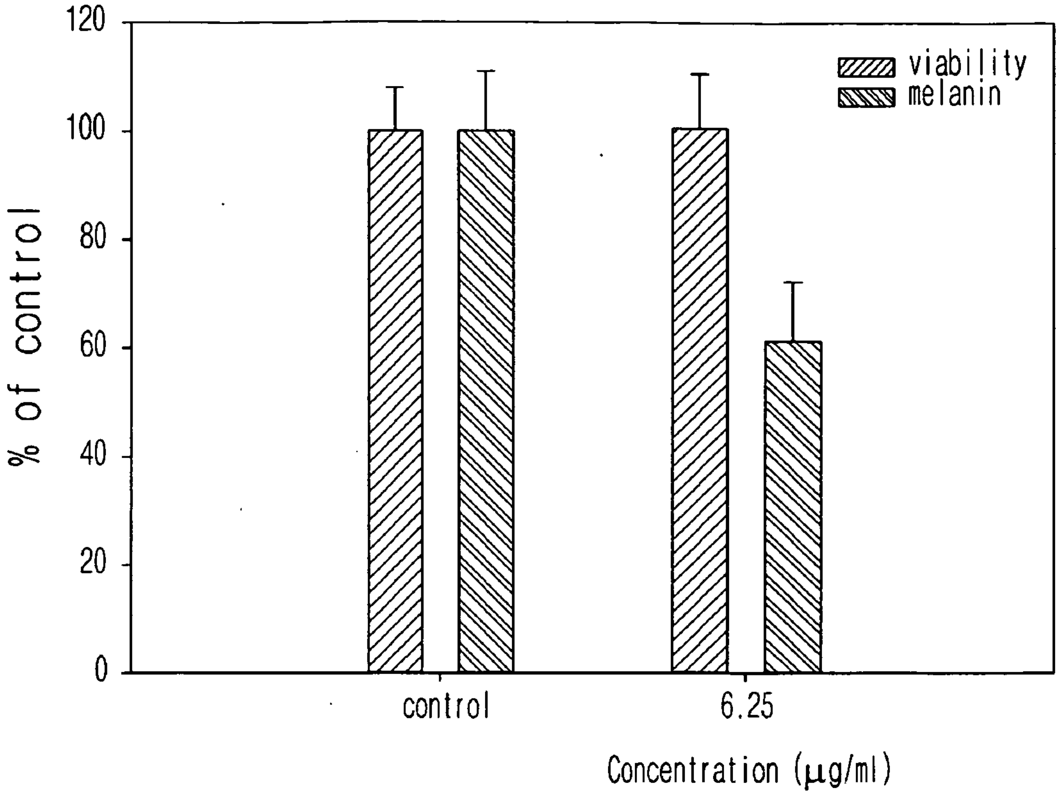
120
100
80
60
40
20
0
% of control
viability
melanin
control
6.25
Concentration (μg/ml)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020080016748
- JP S5544375 B
- JP S6426507 B
- JP S6483009 B
- JP H125687 B
- JP H5139950 B
- KR 92002109
- KR 97021273
- JP S60214721 B
- JP S60214728 B
- JP S6323809 B
- JP S6463506 B
- JP H1149706 B
- KR 97025601
- JP S61246109 B
- KR 92002111
- JP S6330403 B
- JP H5139954 B
- KR 92002110
- JP S60214727 B
- JP S6416709 B
- JP S5692211 B
- JP H426610 B
- JP S6150915 B
- JP H2207028 B
- KR 96033442
- JP S5244375 B
- JP H2207030 B
- KR 100602684